# EUROPÄISCHE PATENTSCHRIFT

(11) **EP 4 563 107 B1**
(45) Veröffentlichungstag und Bekanntmachung des Hinweises auf die Patenterteilung: **12.08.2026**
(21) Anmeldenummer: 24215653.7
(22) Anmeldetag: 27.11.2024
(51) Int. Cl.: A61B 17/88, B21D 7/06, A61B 17/28

(54) **MEDIZINISCHE STABBIEGEZANGE MIT DOPPELGELENK**
MEDICAL ROD BENDING PLIERS WITH DOUBLE JOINT
PINCE À CINTRER À TIGE MÉDICALE À DOUBLE ARTICULATION

(30) Priorität: 28.11.2023 DE 102023133228
(43) Veröffentlichungstag der Anmeldung: 04.06.2025
(73) Patentinhaber: Aesculap AG, 78532 Tuttlingen (DE)
(72) Erfinder: LINKE, Ralph, 78234 Engen (DE); KAHL, Benjamin, 87257 Sonthofen (DE)
(74) Vertreter: Winter, Brandl - Partnerschaft mbB

(56) Entgegenhaltungen:
- DE-A1- 3 119 550
- US-A- 5 490 409
- US-A1- 2017 042 597
- US-A1- 2023 355 289

## Beschreibung

### Technischer Hintergrund

Die vorliegende Erfindung betrifft eine medizinische Stabbiegezange mit einem ersten Gelenk zwischen Griffen und Schwenkarmen mit Biegerollen.

Bei Wirbelsäulenoperationen werden Pedikelschrauben in einzelne Wirbel eingeschraubt und durch einen Stab miteinander verbunden. Somit können einzelne Wirbel, die relativ zu anderen Wirbeln verrutscht sind, sogenannte Gleitwirbel, in einer gewünschten Position fixiert werden. Der Stab, der die Pedikelschrauben verbindet, muss dabei an die Form der Wirbelsäule des jeweiligen Patienten angepasst werden. Es ist bekannt, zum Biegen des Stabes Zangen einzusetzen. Diese Zangen werden auch Stabbiegezangen genannt. Bekannte Stabbiegezangen haben zwei zusammendrückbare Griffe (Griffbranchen), zwei bewegbare Maulteile mit (Stab-)Biegerollen an deren freie Enden und ein Gelenk/Scharnier zwischen dem Griffpaar und den Biegerollen. Jede Griffbranche ist dabei fest mit dem zugehörigen Maulteil vorzugsweise (stoff-) einstückig verbunden und bildet somit ein Zangenbauteil. Beide Zangenbauteile sind nicht scherenförmig sondern wippenartig über das Scharnier miteinander gekoppelt, derart, dass bei einem Zusammendrücken der Griffbranchen die beiden Maulteile auseinanderverschwenkt werden. Dabei folgen die Biegerollen einer Kreisbahn in Richtung hin zu den Griffbranchen.

Die Stabbiegezange weist ferner eine mittlere Auflagefläche (Biegebolzen) auf, die zwischen den beiden Biegerollen sowie in Richtung hin zu dem Gelenk/Scharnier angeordnet ist. Wird das Griffpaar zusammengedrückt, schwenken die Biegerollen derart um das Gelenk/Scharnier, dass sich die Biegerollen auseinander sowie nach hinten zu den Griffen bewegen. Liegt der Stab an der mittleren Auflagefläche an, wird der Stab durch die kreisförmige Bewegung der Biegerollen um die Anlagefläche gebogen. Der Biegeradius des Stabes hängt dabei von der Geometrie der mittleren Auflagefläche ab, wohingegen der Biegewinkel (Maß der Biegebewegung) besonders von einem Verfahrweg oder Bewegungsradius der beiden Biegerollen abhängig ist. Dabei bedeutet, dass ein großer Verfahrweg der Biegerollen einen großen Biegewinkel bewirkt. Der Biegeradius im Bereich der Biegestelle (abhängig von der Anlagefläche) bleibt indessen vom Biegewinkel (Winkelmaß der Abbiegung) unbeeinflusst.

Da die beiden Biegerollen über die Maulteile mechanisch mit den Griffen gekoppelt sind, bedeutet ein großer Verfahrweg der Biegerollen gleichzeitig einen großen Öffnungswinkel der beiden Griffe. Durch den notwendigen, großen Öffnungswinkel der Griffe/Griffbranchen muss die Stabbiegezange von einem Benutzer bzw. Chirurgen häufig mit zwei Händen ähnlich einer Heckenschere bedient oder gehalten werden. Da der Benutzer zusätzlich zur Stabbiegezange aber auch noch den Stab halten muss, wird bei bekannten Stabbiegezangen in der Praxis ein Griff an der Brust des Benutzers als Widerlager angelegt, mit der einen Hand der Stab gehalten und mit der anderen Hand der andere Griff betätigt. Das ist zum einen unergonomisch und kann zum anderen die Stabbiegezange verunreinigen oder unsteril machen.

Aus der US 2017 / 042 597 A1 und der US 2023 / 355 289 A1 ist jeweils eine Stabbiegezange bekannt, bei der zwei Griffe um ein erstes Gelenk schwenkbar sind und zwei Schwenkarme jeweils durch ein zweites Gelenk beweglich mit den jeweiligen Griffen verbunden sind. Die beiden Schwenkarme rotieren jeweils um eine Umlenkrolle. Die US 5 490 409 A offenbart eine Stabbiegezange, bei der zwei Griffe um ein Gelenk schwenkbar sind.

### Zusammenfassung der Erfindung

Es ist die Aufgabe der vorliegenden Erfindung, die Nachteile des Standes der Technik zu überkommen oder zumindest zu mindern und insbesondere eine medizinische Stabbiegezange bereitzustellen, die von einem Benutzer mit lediglich einer Hand gehalten und bedient werden kann. Insbesondere soll der Benutzer beide Griffe der Stabbiegezange mit einer Hand greifen können.

Diese Aufgabe wird erfindungsgemäß durch eine medizinische Stabbiegezange mit den Merkmalen des Anspruchs 1 gelöst. Ferner wird die Aufgabe der vorliegenden Erfindung durch ein System mit den Merkmalen des Anspruchs 12 gelöst

Vorteilhafte Weiterbildungen der vorliegenden Erfindung sind Gegenstand der beigefügten Unteransprüche.

### Zusammenfassung der Offenbarung

Der Grundgedanke der Offenbarung besteht im Wesentlichen darin, die Stabbiegezange als Doppelgelenkzange auszubilden und mit einen Ausleger auszustatten, der sich an dem ersten Gelenk/Scharnier (vergleichbar zu dem Wippenscharnier des vorstehend beschriebenen Stands der Technik) des Doppelgelenkmechanismus abstützt und sich in Richtung hin zu einem Scherenscharnier/Verbindungsstift (in Zangenlängsrichtung) erstreckt an welchem die (Bumerang-artig gebogenen) Maulteile scherenartig miteinander gekoppelt sind. An dem Ausleger ist die aus dem Stand der Technik ebenfalls bekannte mittlere Auflagerfläche ausgebildet oder montiert/montierbar. Der Ausleger ist ferner mit einer Führungskulisse, beispielsweise einen Längsschlitz ausgeformt, die dafür vorgesehen und ausgebildet ist, das Scherenscharnier/Verbindungsstift im Bereich der Maulteile längsbeweglich zu führen.

Die vorliegende Offenbarung betrifft konkreter ausgedrückt eine medizinische Stabbiegezange mit
- zusammendrückbaren Griffen oder Griffschenkeln,
- (Bumerang-artig gebogene) Schwenkarmen / Branchen / Maulteilen, die bei einer Bewegung der Griffe in entgegengesetzte Richtungen bewegbar sind,
- einem Umlenker mit einer Auflagefläche, die dafür vorbereitet und eingerichtet ist, dass der Stab an der Auflagefläche anliegt und an der Auflagefläche biegbar ist, und
- einem ersten (Dreh-)Gelenk/Scharnier, an dem die Griffe zueinander schwenkbar nach Art einer Wippe angeordnet sind. D.h. das erste (Dreh-) Gelenk/Scharnier ist in einem vorderen Endbereich der Griffbranchen angeordnet, derart, dass ein vergleichsweise kurzer Grifffortsatz über das erste (Dreh-)Gelenk/Scharnier hin zu den Maulteilen vorragt.
- Jeder Schwenkarm/Maulteil weist an seinem freien (den Grifffortsätzen abgewandten) Endabschnitt jeweils eine (Stab-)Biegerolle auf, die (zusammen) mit dem jeweiligen Schwenkarm/Maulteil bewegbar ist und dafür vorbereitet und ausgebildet ist, einen Stab (eines Wirbelsäulenimplantats/Pedikelschraube) zu biegen, der in die Stabbiegezange eingelegt ist.
- Die Schwenkarme/Maulteile sind in deren Mittenbereichen mittels eines Gelenks/Verbindungsstifts scherenartig miteinander gekoppelt und an ihren, den Grifffortsätzen zugewandten Enden/Endabschnitten jeweils durch ein zweites (Dreh-)Gelenk/Scharnier an den Grifffortsätzen anscharniert.

Die Schwenkarme sind durch einen Verbindungsstift beweglich aneinander befestigt, der jeweils in einer Bohrung der Schwenkarme gelagert ist. Der Verbindungsstift ist in einer Führungsnut derart beweglich gelagert, dass der Verbindungsstift einen Freiheitsgrad in Richtung einer Längserstreckung der Stabbiegezange hat.

Die Schwenkarme können insbesondere durch einen Verbindungsstift / Verbindungspin / Verbindungsbolzen scherenartig aneinander befestigt sein. Dazu können sich die beiden Schwenkarme in dem Mittelabschnitt kreuzen. Die beiden Schwenkarme können jeweils eine mittlere Bohrung aufweisen, in der der Verbindungsstift gelagert ist. Die Schwenkarme sind jeweils um den Verbindungsstift drehbar oder beweglich gelagert. Der Verbindungsstift kann die beiden gekreuzten Mittelabschnitte (beweglich) verbinden.

Die Schwenkarme können als flache längliche Platte ausgebildet sein, wobei die mittlere Bohrung für den Verbindungsstift jeweils in dem Mittelabschnitt des Schwenkarms angeordnet sein kann. Die Schwenkarme können ferner gebogen sein, sodass sie die Form eines Bumerangs haben. Die Schwenkarme können jeweils eine proximale Gelenkbohrung in dem proximalen Endabschnitt aufweisen, in der der Gelenkstift des zweiten Gelenks gelagert ist. Der Gelenkstift des zweiten Gelenks kann auch in der zweiten Gelenkbohrung am jeweiligen Griffs drehbar gelagert sein.

Die Bewegung der Schwenkarme und insbesondere die Relativbewegung der Schwenkarme zueinander kann zwei Komponenten haben. Die Schwenkarme können jeweils eine rotatorische Bewegung um das zweite Gelenk ausführen, mit dem der jeweilige Schwenkarm an dem Griff befestigt ist. Ferner kann ein Verbindungsabschnitt, in dem die beiden Schwenkarme aneinander befestigt sind, eine translatorische Bewegung in Richtung einer Längserstreckung der Stabbiegezange ausführen. D.h. der Verbindungsstift, der die Schwenkarme verbindet, kann sich axial entlang der Längserstreckung der Stabbiegezange bewegen.

Die rotatorische Bewegung der Schwenkarme kann eine Bewegung des distalen Endabschnitts und damit der Biegerollen auf einem Kreisbogen bewirken. Ist der Stab in die Stabbiegezange eingelegt, können die Biegerollen bei Betätigung / beim Zusammendrücken der Griffe an dem Stab entlangrollen / abrollen. Durch die Bewegung entlang des Kreisbogens und damit der Bewegung relativ zu dem fixierten Stab können die Biegerollen den Stab biegen. Vorzugsweise können die Biegerollen also dafür vorbereitet und eingerichtet sein, den Stab des Wirbelsäulenimplantats zu biegen oder plastisch zu verformen.

Durch die Bewegung des Verbindungsstifts in Richtung der Längserstreckung der Stabbiegezange kann die translatorische Bewegung des Verbindungsstifts durch die Führungsnut geführt sein. Der Verbindungsstift kann insbesondere einen breiten Stiftkopf aufweisen, der in der Führungsnut gelagert oder geführt ist. Ein längerer Stiftzylinder des Verbindungsstiftes, der aus dem Stiftkopf hervorsteht, kann in den mittleren Bohrungen der Schwenkarme gelagert sein. Die Führungsnut kann somit die (translatorische) Bewegung des Verbindungsstifts und damit der Schwenkarme auf einen (translatorischen) Freiheitsgrad eingrenzen. Durch die geführte Bewegung kann eine symmetrische / gleichförmige Bewegung der beiden Schwenkarme bewirkt werden.

In anderen Worten hat die Stabbiegezange zusammendrückbare Griffe (Griffbranchen) bzw. ein zusammendrückbares Griffpaar, das um das erste Gelenk/Scharnier des Doppelgelenkmechanismus nach Art einer Wippe (also nicht scherenartig) zueinander schwenkbar angeordnet ist, wobei jede Griffbranche einen (stoffeinstückigen) Grifffortsatz hat, der über das erste Gelenk/Scharnier hinaus hin zu den Zangenmaulteilen vorragt. Wenn daher die Griffe um das erste Gelenk des Doppelgelenkmechanismus zusammengedrückt werden, bewegen sich die freien Enden der Grifffortsätze um das erste Gelenk auseinander. An jedem der Grifffortsätze ist eines der beiden (Bumerang-artig gebogenen) Maulteile über das zweite Gelenk/Scharnier des Doppelgelenkmechanismus schwenkgekoppelt, wobei die beiden Maulteile in deren jeweiligen Längsmittenabschnitten über das Scherengelenk/Verbindungsstift scherenartig gekoppelt sind.

Ein Zusammendrücken des Griffpaars bewirkt daher ein Auseinanderbewegen der Grifffortsätze zusammen mit den daran gekoppelten Enden der Maulteile. Dies wiederum bewirkt eine (Kreisbahn-)Bewegung der freien Maulteilenden und somit der Biegerollen (infolge der Bumerang-artigen Biegung) in Richtung hin zu den Griffbranchen. D.h. das erste Gelenk bleibt statisch (ortsfest), während die Griffe eine Relativbewegung / Schwenkbewegung um das erste Gelenk ausführen. Durch die Bewegung der Griffe können die Schwenkarme/Maulteile, die (beweglich/scharnierartig) mit den Griffen verbunden sind, bewegt werden. Die Verbindung zwischen den Schwenkarmen und den Griffen stellt das zweite Gelenk dar. Die Bewegung der scherenartig miteinander gekoppelten Schwenkarme/Maulteile bewirkt gleichzeitig eine Bewegung der Biegerollen, die an den Schwenkarmen/Maulteilen befestigt sind. Die Biegerollen bewegen sich somit relativ zu dem an der Anlagefläche gehaltenen Stab auf diesen zu und üben eine Biegekraft beidseits der Anlagefläche auf diesen aus, was zu einer Stab-Biegebewegung führt. Dabei können die Biegerollen an dem Stab abrollen und den Stab durch ihre Kreisbahn plastisch deformieren.

Die Stabbiegezange kann insbesondere eine medizinische Zange zum Biegen von Stäben von Wirbelsäulenimplantaten sein. Durch das Biegen kann der Stab insbesondere an eine anatomische Form eines Patienten und besonders der Wirbelsäule des Patienten angepasst werden.

Das erste Gelenk kann einen ersten Gelenkstift aufweisen, der jeweils in einer ersten Gelenkbohrung an dem jeweiligen Griff drehbar gelagert ist. Das zweite Gelenk kann einen zweiten Gelenkstift aufweisen, der in einer zweiten Gelenkbohrung an dem jeweiligen Griff und einer weiteren Gelenkbohrung in dem jeweiligen Schwenkarm gelagert ist.

Der Zweck der Offenbarung besteht also darin, eine (mechanische) Übersetzung zwischen den Griffen und den Schwenkarmen/Maulteilen mit den Biegerollen bereitzustellen. Diese Übersetzung kann dadurch verwirklicht werden, dass die Verbindung/Kopplung zwischen den Griffen und den Schwenkarmen als Doppelgelenk ausgebildet ist.

Die offenbarungsgemäße Stabbiegezange hat die folgenden Vorteile:
Durch das Doppelgelenk kann ein Verfahrweg der Schwenkarme und damit der Biegerollen von einem Verfahrweg der Griffe und damit einem Öffnungswinkel der Griffe entkoppelt werden. Somit kann der Öffnungswinkel der Griffe vermindert werden, ohne den Verfahrweg der Biegerollen zu vermindern. Die Griffe können also derart zueinander angestellt werden, dass ein Benutzer beide Griffe mit einer Hand greifen kann, auch wenn die Griffe maximal zueinander beabstandet sind. Trotz reduziertem Öffnungswinkel kann der gewünschte Verfahrweg der Biegerollen gewährt werden und somit der gewünschte Biegeradius des Stabes gesichert werden. Der Benutzer kann die Stabbiegezange somit mit einer Hand halten und bedienen. So hat der Benutzer die andere Hand frei, um beispielsweise den zu biegenden Stab zu halten. Somit ist eine ergonomische und sterile Arbeitsweise des Benutzers gewährleistet.

Nach einem optionalen Merkmal der vorliegenden Offenbarung kann die Biegerolle an einem distalen Endabschnitt des jeweiligen Schwenkarms angeordnet sein. Die Schwenkarme können jeweils einen proximalen Endabschnitt, den distalen Endabschnitt und einen Mittelabschnitt aufweisen, der zwischen dem proximalen Endabschnitt und dem distalen Endabschnitt angeordnet ist. Die Biegerolle kann insbesondere drehbar auf dem distalen Endabschnitt angeordnet sein und sich um eine Rotationsachse drehen, die vorzugsweise senkrecht zu einer Längserstreckung der Stabbiegezange angeordnet ist.

Eine distale Richtung kann als zu den Biegerollen zeigend und eine proximale Richtung als in Richtung der Griffe zeigend definiert werden.

Vorzugsweise kann die Führungsnut in einem Führungsabschnitt / Führungselement / Ausleger / Auflageplatte ausgebildet sein. Der Führungsabschnitt kann dabei einen flachen plattenförmigen Grundkörper aufweisen. An einer proximalen Seite des plattenförmigen Grundkörpers kann eine Bohrung ausgebildet sein, in der der erste Gelenkstift des ersten Gelenks gelagert ist. Der Führungsabschnitt kann also durch das erste Gelenk positionsbestimmt gelagert sein. Auf einer distalen Seite des plattenförmigen Grundkörpers kann die längliche Führungsnut ausgebildet sein. Die Tiefe der Führungsnut kann dabei einer Dicke des breiten Stiftkopfes des Verbindungsstifts entsprechen. Der Stiftzylinder des Verbindungsstifts kann in einer länglichen Aussparung des Führungsabschnitts geführt sein. Der plattenförmige Grundkörper kann insbesondere die Form einer Gabel oder eines Schraubenschlüssels aufweisen.

Die Stabbiegezange hat einen Umlenker / ein Handrad / einen Adjuster / einen Aktor / eine Einstellvorrichtung. Der Umlenker kann eine Auflagefläche bzw. eine Biegefläche aufweisen, die dafür vorbereitet und eingerichtet sein kann, an dem Stab anzuliegen und den Stab zu biegen. Zusammen mit den beiden Biegerollen kann die Auflagefläche somit (zumindest) drei Auflageflächen oder -punkte bilden, an denen der Stab aufliegen kann. Der Umlenker kann in Breitenrichtung der Stabbiegezange gesehen zwischen den Biegerollen angeordnet sein. D.h. der Umlenker kann auf einer Symmetrieachse / Längsachse der Stabbiegezang angeordnet sein, während die Biegerollen jeweils von der Längsachse aus nach außen versetzt sind. Somit kann der Stab durch die Relativbewegung der beiden Biegerollen um die Auflagefläche des Umlenkers gebogen werden. Somit kann der Umlenker eine Funktion eines Umlenk- oder Anlageelements aufweisen.

Vorzugsweise kann der Umlenker auf einer Aufnahmewelle des Führungsabschnitts gelagert sein. Die Aufnahmewelle kann dabei an einem Führungsabschnitt angeordnet sein oder aus dem Führungsabschnitt herausragen. Insbesondere kann die Aufnahmewelle als ein zylinderförmiger Aufsatz ausgebildet sein, der senkrecht aus dem plattenförmigen Grundkörper des Führungsabschnitts herausragt. D.h. die Längsachse der Aufnahmewelle kann sich senkrecht zur Längserstreckung der Stabbiegezange erstrecken. Die Aufnahmewelle kann dabei insbesondere in einer Längsrichtung des Führungsabschnitts gesehen mittig angeordnet sein. Der Führungsabschnitt kann somit eine Auflage bzw. ein Auflageelement für den Umlenker darstellen.

Nach einem weiteren optionalen Merkmal der vorliegenden Offenbarung kann der Umlenker um die Aufnahmewelle drehbar gelagert sein. D.h. der Umlenker kann um eine Rotationsachse drehbar sein, die der Längsachse der Aufnahmewelle entspricht.

Vorzugsweise kann der Umlenker derart ausgebildet sein, dass der Umlenker nicht rotationssymmetrisch ist. Insbesondere kann der Umlenker je nach Ausrichtung bzw. Drehrichtung verschiedene Auflageflächen mit unterschiedlich großen Kontaktflächen oder Krümmungsradien ausbilden. Die Ausgestaltung der Auflageflächen kann den Biegeradius oder eine Biegekontur des Stabs beeinflussen. Insbesondere kann ein Abstand zwischen den Biegerollen und einem Scheitelpunkt der Auflagefläche des Umlenkers, in der Längserstreckung der Stabbiegezange gesehen, die Biegung des Stabes beeinflussen. Da durch die Krümmung der Auflagefläche auch der Scheitelpunkt variiert werden kann, kann die Biegung des Stabes auch von der Krümmung der Auflageflächen abhängen.

Vorzugsweise kann der Umlenker entlang der Aufnahmewelle axial beweglich bzw. in Axialrichtung der Aufnahmewelle verschiebbar gelagert sein. D.h. der Umlenker kann senkrecht zu der Längserstreckung der Stabbiegezange bewegbar sein. Somit kann der Umlenker insgesamt zwei Freiheitsgrade aufweisen, nämlich einen translatorischen und einen rotatorischen Freiheitsgrad. Durch die Bewegung entlang der Aufnahmewelle kann der Umlenker von dem Führungsabschnitt abgehoben werden. Durch gleichzeitiges Anheben und Drehen des Umlenkers, insbesondere durch den Benutzer, kann der Umlenker in unterschiedliche Stellungen gedreht werden.

Der Umlenker kann insbesondere als ein runder Drehknopf ausgebildet sein. Der Umlenker kann dabei derart ausgestalten sein, dass ein Rand- oder Greifabschnitt des Umlenkers von dem Benutzer greifbar ist. Der Benutzer kann den Umlenker somit sowohl um die Rotationsachse drehen als auch entlang der Aufnahmewelle verschieben.

Nach einem weiteren optionalen Merkmal der vorliegenden Offenbarung kann eine Feder zwischen dem Umlenker und der Aufnahmewelle bereitgestellt sein, deren Federkraft in axialer Richtung der Aufnahmewelle wirkt. Die Feder kann vorzugsweise an einem Überstand der Aufnahmewelle befestigt sein. Wenn der Benutzer den Umlenker von dem Führungsabschnitt wegzieht bzw. abhebt, wird die Feder gestaucht. Lässt der Benutzer den Umlenker los, wird er durch die Federkraft zurück zu dem Führungsabschnitt geschoben. Die Feder kann beispielsweise eine Schraubenfeder sein.

Der Überstand der Aufnahmewelle kann insbesondere durch eine überstehende Kappe / Wellenabschlussbolzen auf der Welle bzw. einen (überstehenden) Wellenabschluss gebildet werden. Der Überstand kann auch verhindern, dass der Umlenker von der Aufnahmewelle abgeleitet.

Vorzugsweise kann der Führungsabschnitt einen vorstehenden Zapfen aufweisen, der abhängig von einer Ausrichtung des Umlenkers in entsprechende
(Arretierungs-) Aufnahmen des Umlenkers eingreift. Vorzugsweise kann der Umlenker drei Aufnahmen haben. Dadurch kann der Umlenker in drei verschiedenen Ausrichtungen arretiert oder festgestellt werden. In jeder der unterschiedlichen Ausrichtung kann eine andere Auflagefläche in distale Richtung also in Richtung der Biegerollen zeigen. Der vorstehende Zapfen kann vorzugsweise neben der Aufnahmewelle ausgebildet sein.

Die Aufnahmen am Umlenker können insbesondere in einer Anlagefläche des Umlenkers ausgebildet sein, mit der der Umlenker auf dem Führungsabschnitt aufliegt. Ferner kann der Umlenker in eine Reinigungsstellung gedreht werden. In der Reinigungsstellung kann der Zapfen derart auf der Auflagefläche des Umlenkers anliegen, dass der Umlenker durch den Zapfen von dem Führungsabschnitt beabstandet ist. D.h. der Benutzer kann den Umlenker von dem Führungsabschnitt anheben und den Umlenker um die Rotationsachse soweit verdrehen, bis der Zapfen auf der Auflagefläche aufliegt. Somit kann der Umlenker in der Reinigungsstellung von dem Führungsabschnitt beabstandet sein. Dadurch kann eine Reinigungsflüssigkeit in den Raum zwischen dem Umlenker und dem Führungsabschnitt eindringen und den Raum reinigen und/oder desinfizieren.

Vorteilhafterweise kann die Feder an einer Außenseite der Aufnahmewelle positioniert sein. Somit kann die Feder für die Reinigungsflüssigkeit zugänglich sein.

Vorzugsweise kann der Umlenker Aussparungen / Löcher in einer Seitenwand aufweisen. Durch die Aussparungen kann die Reinigungsflüssigkeit in das Innere des Umlenkers eindringen und die Aufnahmewelle und/oder die Feder erreichen.

Die Aufgabe der vorliegenden Offenbarung wird ferner durch ein System aus dem Stab für das Wirbelsäulenimplantat und der Stabbiegezange nach einem der vorstehenden Aspekte gelöst.

### Kurzbeschreibung der Figuren

Fig. 1 zeigt eine perspektivische Ansicht einer Stabbiegezange nach der vorliegenden Offenbarung;
Fig. 2 zeigt eine Unterseite der Stabbiegezange nach der vorliegenden Offenbarung;
Fig. 3 zeigt eine Draufsicht auf einen Schwenkarm der Stabbiegezange nach der vorliegenden Offenbarung;
Fig. 4 zeigt eine Draufsicht auf die Stabbiegezange nach der vorliegenden Offenbarung;
Fig. 5 zeigt eine perspektivische Ansicht eines Führungsabschnitts der Stabbiegezange nach der vorliegenden Offenbarung;
Fig. 6 zeigt eine Seitenansicht eines Verbindungsstifts der Stabbiegezange nach der vorliegenden Offenbarung;
Fig. 7 zeigt eine perspektivische Ansicht eines Drehknopfs der Stabbiegezange nach der vorliegenden Offenbarung;
Fig. 8 zeigt einen Längsschnitt durch den Drehknopf in Fig. 7;
Fig. 9 zeigt eine schematische Ansicht einer Feder der Stabbiegezange nach der vorliegenden Offenbarung; und
Fig. 10 zeigt eine perspektivische Ansicht eines Griffs der Stabbiegezange nach der vorliegenden Offenbarung.

### Detaillierte Beschreibung der Figuren

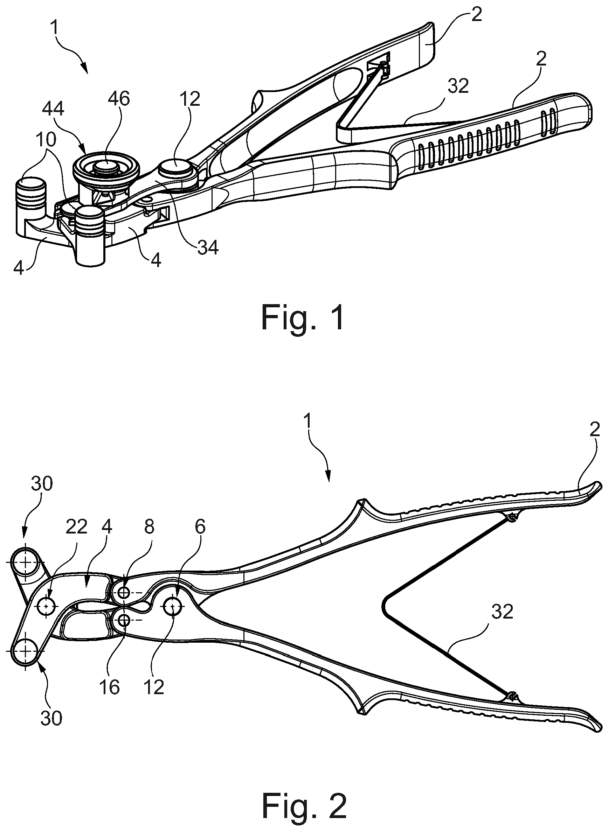

Fig. 1 zeigt eine Stabbiegezange 1. Die Stabbiegezange 1 hat zwei zusammendrückbare Griffe 2 und zwei Schwenkarme 4. Die Griffe 2 sind um ein erstes (Dreh-)Gelenk 6 gegeneinander verschwenkbar. Die Schwenkarme 4 sind jeweils durch ein zweites (Dreh-)Gelenk 8 beweglich mit den jeweiligen Griffen 2 verbunden. Die Schwenkarme 4 weisen jeweils eine Biegerolle 10 auf. Die Biegerollen 10 sind jeweils um eine Rotationsachse drehbar, die senkrecht zu einer Längserstreckung der Stabbiegezange 1 ausgerichtet ist. Dadurch können die Biegerollen 10 bei einer Bewegung an einem Stab (nicht dargestellt) abrollen. Im Folgenden wird distal als zu den Biegerollen 10 zeigend und proximal als in Richtung der Griffe 2 zeigend definiert.

Fig. 2 zeigt eine Unterseite der Stabbiegezange 1. Das erste Gelenk 6 hat einen ersten Gelenkstift 12, der jeweils in einer ersten Gelenkbohrung 14 des jeweiligen Griffs 2 gelagert ist. Das zweite Gelenk 8 verbindet jeweils einen Griff 2 mit einem der Schwenkarme 4 und weist jeweils einen zweiten Gelenkstift 16 auf. Der zweite Gelenkstift 16 ist jeweils in einer zweiten Gelenkbohrung 18 in dem Griff 2 und einer proximalen Gelenkbohrung 20 in dem Schwenkarm 4 gelagert. Die beiden Schwenkarme 4 (über-)kreuzen sich und sind in einem Verbindungsabschnitt durch einen Verbindungsstift 22 schwenk- oder drehbar miteinander verbunden. Der Verbindungsstift 22 ist jeweils in einer mittleren Bohrung 24 in einem Mittelabschnitt 26 der Schwenkarme 4 schwenk- oder drehbar gelagert.

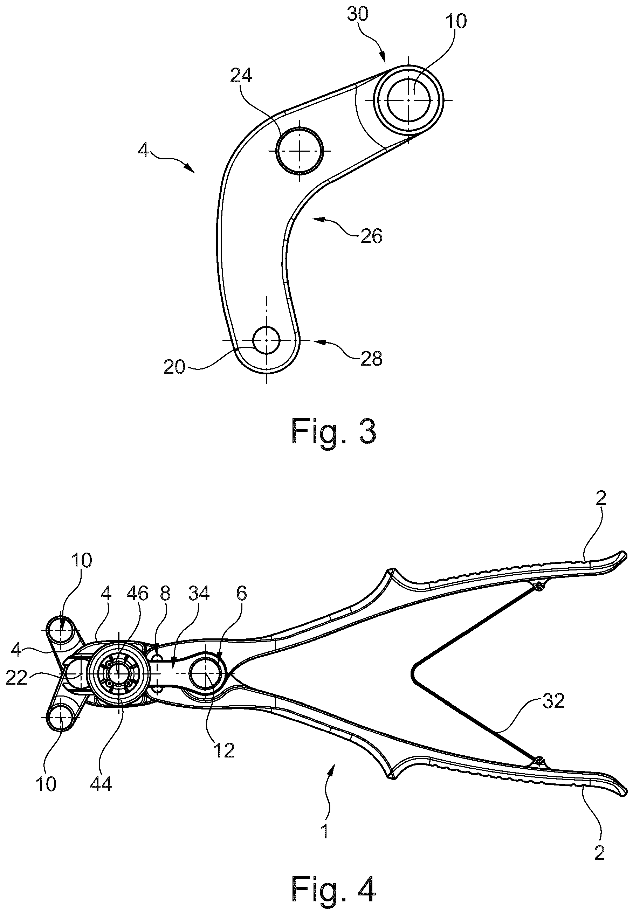

Fig. 3 zeigt eine Draufsicht auf einen der Schwenkarme 4. Die Schwenkarme 4 weisen jeweils einen proximalen Endabschnitt 28 und einen distalen Endabschnitt 30 auf. In dem proximalen Endabschnitt 28 ist die proximale Gelenkbohrung 20 angebracht, in der der zweite Gelenkstift 16 des zweiten Gelenks 8 gelagert ist. In dem distalen Endabschnitt 30 ist die Biegerolle 10 angeordnet oder drehbar befestigt. In dem Mittelabschnitt 26 des Schwenkarms 4, der zwischen dem proximalen Endabschnitt 28 und dem distalen Endabschnitt 30 angeordnet ist, ist die mittlere Bohrung 24 ausgebildet.

Fig. 4 zeigt eine Draufsicht auf die Stabbiegezange 1. Die Stabbiegezange 1 weist ferner eine Auflage(-platte) bzw. einen Ausleger/Führungsabschnitt 34 auf. Der Ausleger / Führungsabschnitt 34 hat im Wesentlichen einen flachen plattenförmigen Grundkörper 36. Auf einer proximalen Seite des plattenförmigen Grundkörpers 36 ist eine Bohrung 38 eingebracht, in der der erste Gelenkstift 12 des ersten Gelenks 6 gelagert ist. Somit ist der Führungsabschnitt 34 an dem ersten Gelenk 6 festgelegt. Auf einer distalen Seite des plattenförmigen Grundkörpers 36 weist der Führungsabschnitt 34 eine längliche Führungsnut 40 auf. Der Verbindungsstift 22, der die beiden Schwenkarme 4 beweglich verbindet, ist in der Führungsnut 40 axial geführt. Aus dem plattenförmigen Grundkörper 36 des Führungsabschnitts 34 steht ferner eine Aufnahmewelle 42 hervor. Auf der Aufnahmewelle 42 ist ein Umlenker / Drehknopf 44 angeordnet. Der Drehknopf 44 ist um die Aufnahmewelle 42 rotierbar und in Axialrichtung der Aufnahmewelle 42 verschiebbar gelagert.

Die Funktionsweise der Stabbiegezange 1 wird nachfolgend beschrieben. Wenn ein Benutzer die beiden Griffe 2 zusammendrückt, schwenken die Griffe 2 um das erste Gelenk 6 mit dem ersten Gelenkstift 12. Dadurch werden die zweiten Gelenke 8 mit den zweiten Gelenkstiften 16, die an dem distalen Ende der Griffe 2 angeordnet sind, nach außen also von einer Symmetrie- oder Längsachse der Stabbiegezange 1 wegbewegt. Die Bewegung der zweiten Gelenke 8 nach außen bedingt eine Schwenkbewegung der Schwenkarme 4. Der proximale Endabschnitt 28 des Schwenkarms 4 schwenkt mit dem zweiten Gelenk 8 nach außen. Der Verbindungsstift 22 zwischen den beiden Schwenkarmen 4 vollzieht eine translatorische Bewegung entlang der Längserstreckung der Stabbiegezange 1. Der distale Endabschnitt 30 und somit die Biegerollen 10 bewegen sich auf einer Kreisbahn. Durch die kreisförmige Bewegung der Biegerollen 10 kann der Stab gebogen werden, der an dem Drehknopf 44 und den beiden Biegerollen 10 anliegt. Die Biegung des Stabes hängt besonders von einem Verfahrweg der Biegerollen 10 auf der Kreisbahn ab. Durch das erste Gelenk 6 und das zweite Gelenk 8 wird eine Übersetzung zwischen einem Öffnungswinkel / Verfahrweg der Griffe 2 und dem Verfahrweg der Biegerollen 10 verwirklicht.

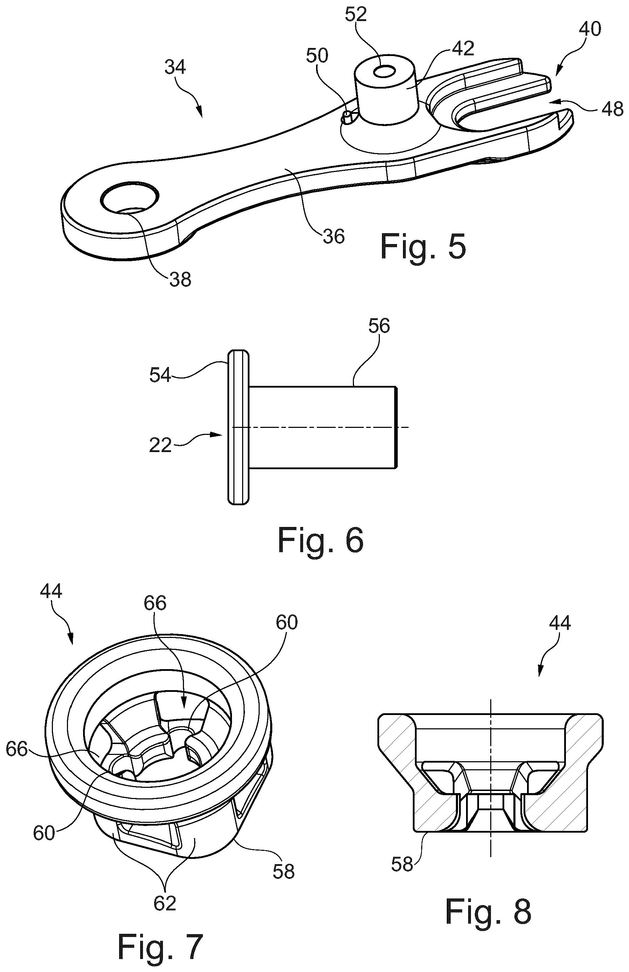

Fig. 5 zeigt eine perspektivische Ansicht des Führungsabschnitts 34 mit dem plattenförmigen Grundkörper 36. Aus dem plattenförmigen Grundkörper 36 steht die Aufnahmewelle 42 hervor, die als zylinderförmiger Vorsprung ausgebildet ist. Der Führungsabschnitt 34 weist die Bohrung 38 auf, in der der erste Gelenkstift 12 gelagert ist. Die Aufnahmewelle 42 ist in Längsrichtung des Grundkörpers 36 gesehen mittig angeordnet und liegt damit wie die Bohrung 38 in einer Symmetrieachse des Grundkörpers 36. Der Führungsabschnitt 34 hat ferner einen vorstehenden Zapfen 50. Der vorstehende Zapfen 50 ist neben der Aufnahmewelle 42 positioniert. Die Aufnahmewelle 42 hat eine axiale Wellenbohrung 52. In die Wellenbohrung 52 kann ein Wellenabschlussbolzen 46 eingesetzt werden. Der Wellenabschlussbolzen 46 kann über einen Durchmesser der Aufnahmewelle 42 hinausstehen. Die Führungsnut 40 weist eine längliche Aussparung 48 auf. Die Führungsnut 40 ist damit derart ausgestaltet, dass ein Stiftkopf 54 des Verbindungsstifts 22 in der Führungsnut 40 axial geführt ist. Ein Stiftzylinder 56, der aus dem Stiftkopf 54 hervorsteht, hat einen kleineren Durchmesser als der Stiftkopf 54. Der Stiftzylinder 56 ragt durch die längliche Aussparung 48 hindurch und ist in dieser axial geführt. Somit hat das erste Gelenk 6 durch die Führungsnut 40 einen translatorischen Freiheitsgrad in Längsrichtung der Stabbiegezange 1. Der translatorische Freiheitsgrad bewirkt eine symmetrische Bewegung der beiden Schwenkarme 4. Fig. 6 zeigt den Verbindungsstift 22 mit dem Stiftkopf 54, der einen größeren Durchmesser aufweist als der Stiftzylinder 56.

Die Figuren 7 und 8 zeigen den Drehknopf 44. Der Drehknopf 44 ist im Wesentlichen rund und um die Aufnahmewelle 42 rotierbar. Der Drehknopf 44 hat eine Anlagefläche 58, die an dem Führungsabschnitt 34 anliegt. In der Anlagefläche 58 sind (Arretierungs-) Aufnahmen 60 ausgebildet, die dafür geeignet oder ausgebildet sind, den Zapfen 50 aufzunehmen. Wenn der Zapfen 50 in die Aufnahme 60 einrastet, ist der drehbare Drehknopf 44 in dieser Stellung verrastet oder arretiert. Eine Seitenwand des Drehknopfs 44 bildet je nach Drehrichtung verschiedene Auflageflächen 62 aus. Das heißt, der Drehknopf 44 ist nicht rotationssymmetrisch, sondern die Konturen der einzelnen Auflageflächen 62 unterscheiden sich. Je nach Stellung kann somit eine andere Geometrie in Richtung der Biegerollen 10 zeigen. Die Auflageflächen 62 sind dafür geeignet, dass der Stab (nicht dargestellt) an ihnen anliegt und durch die Kombination aus Biegerollen 10 und Auflagefläche 62 gebogen wird. Der Drehknopf 44 weist ferner einen Rand auf, den der Benutzer greifen kann. Somit kann der Benutzer den Drehknopf 44 um die Rotationsachse drehen als auch entlang der Aufnahmewelle 42 verschieben. Der Drehknopf 44 ist also in Axialrichtung der Aufnahmewelle 42 von dem Führungsabschnitt 34 abhebbar. Durch eine Kombination aus Abheben des Drehknopfs 44 und gleichzeitiger Rotation um die Aufnahmewelle 42 kann der Drehknopf 44 in die unterschiedlichen Stellungen gedreht oder eingestellt werden. Durch die unterschiedlichen Einstellungen des Drehknopfs 44 kann ein Biegeverhalten oder ein Biegeradius des Stabes variiert werden.

Wenn der Drehknopf 44 derart angehoben und gedreht wird, dass der Zapfen 50 nicht in eine Aufnahme 60 einrastet, sondern auf der Anlagefläche 58 aufliegt, ist die Anlagefläche 58 von dem Grundkörper 36 des Führungsabschnitts 34 beabstandet. Diese Stellung wird als Reinigungsstellung bezeichnet. Durch den Abstand zwischen der Anlagefläche 58 und dem Grundkörper 36 kann Reinigungsflüssigkeit zwischen die jeweiligen Flächen eindringen.

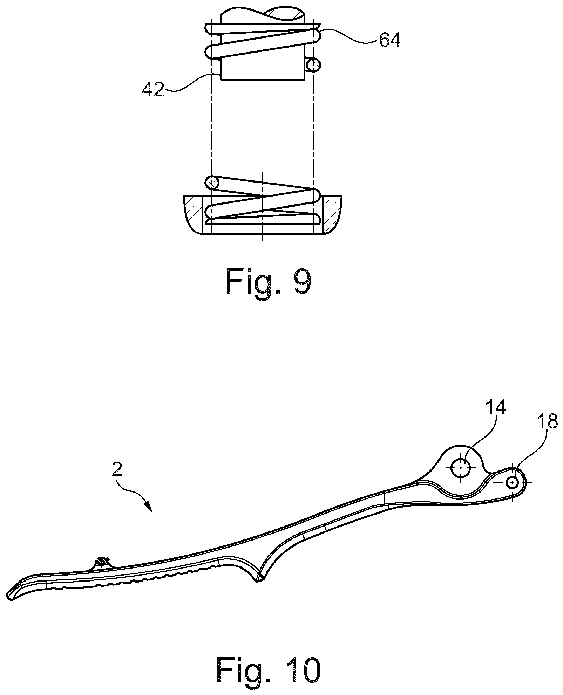

Der Drehknopf 44 ist mit einer Feder 64 vorgespannt. Die Feder 64 ist in der Fig. 9 gezeigt und ist an einer Kante an dem Drehknopf 44 befestigt. Die andere Seite der Feder 64 ist an dem Wellenabschlussbolzen 46 befestigt. Die Feder 64 verläuft entlang einer Außenseite der Aufnahmewelle 42. Somit wirkt eine Federkraft der Feder 64 in Richtung der Aufnahmewelle 42. Wenn der Drehknopf 44 (durch den Benutzer) von dem Führungsabschnitt 34 abgehoben wird, wird die Feder 64 gestaucht. Entfällt die externe Kraft auf den Drehknopf 44, wird der Drehknopf durch die Federkraft zurück in Richtung des Führungsabschnitts 34 geschoben. Somit wird zusätzlich sichergestellt, dass der Drehknopf 44 in den verschiedenen Arretierstellungen einrastet. Der Drehknopf 44 weist ferner Aussparungen / Löcher 66 in der Seitenwand auf. Dadurch kann Reinigungsflüssigkeit besonders gut zu der Aufnahmewelle 42 und der Feder 64 vordringen. Fig. 10 zeigt einen Griff 2 mit einer ersten Gelenkbohrung 14 und einer zweiten Gelenkbohrung 18 für das zweite Gelenk 8.

### Bezugszeichenliste

- 1: Stabbiegezange
- 2: Griff
- 4: Schwenkarm
- 6: erstes Gelenk
- 8: zweites Gelenk
- 10: Biegerolle
- 12: erster Gelenkstift
- 14: erste Gelenkbohrung
- 16: zweiter Gelenkstift
- 18: zweite Gelenkbohrung
- 20: proximale Gelenkbohrung
- 22: Verbindungsstift
- 24: mittlere Bohrung
- 26: Mittelabschnitt
- 28: proximaler Endabschnitt
- 30: distaler Endabschnitt
- 34: Ausleger/Führungsabschnitt
- 36: Grundkörper
- 38: Bohrung
- 40: Führungsnut
- 42: Aufnahmewelle
- 44: Umlenker / Drehknopf
- 46: Wellenabschlussbolzen
- 48: längliche Aussparung
- 50: Zapfen
- 52: Wellenbohrung
- 54: Stiftkopf
- 56: Stiftzylinder
- 58: Anlagefläche
- 60: Aufnahme
- 62: Auflagefläche
- 64: Feder
- 66: Aussparung

## Patentansprüche

1. Medizinische Stabbiegezange (1) mit zusammendrückbaren Griffen (2), die um ein erstes Gelenk (6) schwenkbar angeordnet sind, Schwenkarmen (4), die bei einer Bewegung der Griffe (2) in jeweils entgegengesetzte Richtungen bewegbar sind, und einem Umlenker (44) mit einer Auflagefläche (62),
wobei jeder Schwenkarm (4) jeweils eine Biegerolle (10) aufweist, die mit dem Schwenkarm (4) bewegbar ist und dafür vorbereitet und ausgebildet ist, einen Stab, insbesondere eines Wirbelsäulenimplantats, zu biegen, der in die Stabbiegezange (1) eingelegt ist,
wobei die Auflagefläche (62) dafür vorbereitet und eingerichtet ist, dass der Stab an der Auflagefläche (62) anliegt und an der Auflagefläche (62) biegbar ist, und
wobei die Schwenkarme (4) jeweils durch ein zweites Gelenk (8) beweglich mit den jeweiligen Griffen (2) verbunden sind,
**dadurch gekennzeichnet, dass**
die Schwenkarme (4) durch einen Verbindungsstift (22) beweglich aneinander befestigt sind, der jeweils in einer Bohrung (24) der Schwenkarme (4) gelagert ist, und
wobei der Verbindungsstift (22) in einer Führungsnut (40) derart beweglich gelagert ist, dass der Verbindungsstift (22) einen Freiheitsgrad in Richtung einer Längserstreckung der Stabbiegezange (1) hat.

2. Stabbiegezange (1) nach Anspruch 1, **dadurch gekennzeichnet, dass** die Führungsnut (40) in einem Führungsabschnitt (34) der Stabbiegezange (1) ausgebildet ist.

3. Stabbiegezange (1) nach Anspruch 2, **dadurch gekennzeichnet, dass** der Umlenker (44) auf einer Aufnahmewelle (42) des Führungsabschnitts (34), insbesondere beweglich, gelagert ist.

4. Stabbiegezange (1) nach Anspruch 3, **dadurch gekennzeichnet, dass** der Umlenker (44) um die Aufnahmewelle (42) drehbar gelagert ist.

5. Stabbiegezange (1) nach Anspruch 4, **dadurch gekennzeichnet, dass** der Umlenker (44) nicht rotationssymmetrisch ist und je nach Drehrichtung unterschiedliche Auflageflächen (62) ausbildet, die vorzugsweise einen Radius einer Stabbiegung definieren.

6. Stabbiegezange (1) nach einem der Ansprüche 3 bis 5, **dadurch gekennzeichnet, dass** der Umlenker (44) entlang der Aufnahmewelle (42) axial beweglich gelagert ist.

7. Stabbiegezange (1) nach Anspruch 6, **dadurch gekennzeichnet, dass** eine Feder (64) zwischen dem Umlenker (44) und der Aufnahmewelle (42) bereitgestellt ist, deren Federkraft in axialer Richtung der Aufnahmewelle (42) wirkt.

8. Stabbiegezange (1) nach Anspruch 7, **dadurch gekennzeichnet, dass** die Feder (64) an einem Überstand, insbesondere an einem überstehenden Wellenabschlussbolzen (46), der Aufnahmewelle (42) befestigt ist.

9. Stabbiegezange (1) nach einem der Ansprüche 2 bis 8, **dadurch gekennzeichnet, dass** der Führungsabschnitt (34) einen vorstehenden Zapfen (50) aufweist, der abhängig von einer Ausrichtung des Umlenkers (44) in entsprechende Aufnahmen (60) des Umlenkers (44) eingreift und den Umlenker (44) in einer Reinigungsstellung von dem Führungsabschnitt (34) beabstandet.

10. Stabbiegezange (1) nach einem der Ansprüche 1 bis 9, **dadurch gekennzeichnet, dass** die Biegerollen (10) jeweils an einem distalen Endabschnitt (30) des Schwenkarms (4), insbesondere um eine Rotationsachse drehbar, angeordnet sind.

11. Stabbiegezange (1) nach einem der Ansprüche 1 bis 10, **dadurch gekennzeichnet, dass** der Umlenker (44) Aussparungen (66) in einer Seitenwand des Umlenkers (44) aufweist.

12. System aus einem Stab eines Wirbelsäulenimplantats und einer medizinischen Stabbiegezange (1) nach einem der Ansprüche 1 bis 11.

## Claims

1. Medical rod bender (1) with compressible handles (2) that are pivotally mounted about a first hinge (6), pivot arms (4), which are movable in opposite directions when the handles (2) are moved, and a deflector (44) with a support surface (62),
wherein each pivot arm (4) comprises a bending roller (10) that is movable with the pivot arm (4) and is designed and configured to bend a rod, in particular, a spinal implant, that is inserted into the rod bender (1),
wherein the support surface (62) is designed and configured such that the rod rests against the support surface (62) and can be bent against the support surface (62), and wherein the pivot arms (4) each are movably connected to the respective handles (2) via a second hinge (8),
**characterized in that**
the pivot arms (4) are movably fastened to one another by a connecting pin (22), which is mounted in a respective bore (24) of the pivot arms (4), and
wherein the connecting pin (22) is movably mounted in a guide groove (40) such that the connecting pin (22) has one degree of freedom in the direction of the longitudinal extension of the rod bender (1).

2. Rod-bender (1) according to claim 1, **characterized in that** the guide groove (40) is formed in a guide section (34) of the rod bender (1).

3. Rod bender (1) according to claim 2, **characterized in that** the deflector (44) is mounted on a mounting shaft (42) of the guide section (34), in particular in a movable manner.

4. Rod bender (1) according to claim 3, **characterized in that** the deflector (44) is mounted so as to rotate about the mounting shaft (42).

5. Rod bender (1) according to claim 4, **characterized in that** the deflector (44) is not rotationally symmetric and, depending on the direction of rotation, forms different support surfaces (62) that preferably define the radius of a rod bend.

6. Rod bender (1) according to any of claims 3 through 5, **characterized in that** the deflector (44) is mounted so as to be axially movable along the mounting shaft (42).

7. Rod bender (1) according to claim 6, **characterized in that** a spring (64) is provided between the deflector (44) and the mounting shaft (42), the spring force of which acts in the axial direction of the mounting shaft (42).

8. Rod bender (1) according to claim 7, **characterized in that** the spring (64) is secured to the mounting shaft (42) at a protrusion, in particular at a protruding shaft end pin (46), of the mounting shaft (42).

9. Rod bender (1) according to any of claims 2 through 8, **characterized in that** the guide section (34) has a protruding pin (50) which, depending on the orientation of the deflector (44), engages with corresponding receiving portions (60) of the deflector (44) and spaces the deflector (44) apart from the guide section (34) in a cleaning position.

10. Rod bender (1) according to any of claims 1 through 9, **characterized in that** the bending rollers (10) are each arranged on a distal end section (30) of the pivot arm (4), in particular so as to be rotatable about an axis of rotation.

11. Rod bender (1) according to any of claims 1 through 10, **characterized in that** the deflector (44) has recesses (66) in a side wall of the deflector (44).

12. A system consisting of a spinal implant rod and a medical rod bender (1) according to any of claims 1 through 11.

## Revendications

1. Pince à cintrer médicale (1) avec des poignées repliables (2) qui sont disposées de manière pivotante autour d'une première articulation (6), des bras pivotants (4) qui sont mobiles lors d'un mouvement des poignées (2) dans des directions respectivement opposées et un déviateur (44) avec une surface d'appui (62),
dans laquelle chaque bras pivotant (4) présente respectivement un rouleau de cintrage (10) qui est mobile avec le bras pivotant (4) et est préparé et réalisé pour plier une tige, en particulier un implant de colonne vertébrale, qui est insérée dans la pince à cintrer (1),
dans laquelle la surface d'appui (62) est préparée et conçue de sorte que la tige repose au niveau de la surface d'appui (62) et est mobile au niveau de la surface d'appui (62), et
dans laquelle les bras pivotants (4) sont respectivement reliés de manière mobile avec les poignées respectives (2) par le biais d'une seconde articulation (8),
**caractérisée en ce que**
les bras pivotants (4) sont fixés les uns aux autres de manière mobile par le biais d'une goupille de liaison (22) qui est logée respectivement dans un alésage (24) des bras pivotants (4), et
dans laquelle la goupille de liaison (22) est logée de manière mobile dans une rainure de guidage (40) de telle sorte que la goupille de liaison (22) présente un degré de liberté en direction d'une étendue longitudinale de la pince à cintrer (1).

2. Pince à cintrer (1) selon la revendication 1, **caractérisée en ce que** la rainure de guidage (40) est réalisée dans une section de guidage (34) de la pince à cintrer (1).

3. Pince à cintrer (1) selon la revendication 2, **caractérisée en ce que** le déviateur (44) est logé, en particulier de manière mobile, sur un arbre d'admission (42) de la section de guidage (34).

4. Pince à cintrer (1) selon la revendication 3, **caractérisée en ce que** le déviateur (44) est logé de manière mobile autour de l'arbre d'admission (42).

5. Pince à cintrer (1) selon la revendication 4, **caractérisée en ce que** le déviateur (44) n'est pas symétrique en rotation et, selon la direction de rotation, réalise différentes surfaces d'appui (62) qui définissent de préférence un rayon d'un cintrage.

6. Pince à cintrer (1) selon l'une quelconque des revendications 3 à 5, **caractérisée en ce que** le déviateur (44) est logé de manière mobile axialement le long de l'arbre d'admission (42).

7. Pince à cintrer (1) selon la revendication 6, **caractérisée en ce qu'**un ressort (64) est mis à disposition entre le déviateur (44) et l'arbre d'admission (42), dont la force élastique agit dans la direction axiale de l'arbre d'admission (42).

8. Pince à cintrer (1) selon la revendication 7, **caractérisée en ce que** le ressort (64) est fixé au niveau d'une projection, en particulier au niveau d'un boulon d'extrémité d'arbre (46) en saillie, de l'arbre d'admission (42).

9. Pince à cintrer (1) selon l'une quelconque des revendications 2 à 8, **caractérisée en ce que** la section de guidage (34) présente un tenon saillant (50) qui s'engrène en fonction d'une orientation du déviateur (44) dans des admissions (60) correspondantes du déviateur (44) et maintient le déviateur (44) à distance de la section de guidage (34) dans une position de nettoyage.

10. Pince à cintrer (1) selon l'une quelconque des revendications 1 à 9, **caractérisée en ce que** les rouleaux de cintrage (10) sont respectivement disposés au niveau d'une section d'extrémité distale (30) du bras pivotant (4), en particulier de manière rotative autour d'un axe de rotation.

11. Pince à cintrer (1) selon l'une quelconque des revendications 1 à 10, **caractérisée en ce que** le déviateur (44) présente des évidements (66) dans une paroi latérale du déviateur (44).

12. Système constitué d'une tige d'un implant de colonne vertébrale et d'une pince à cintrer médicale (1) selon l'une quelconque des revendications 1 à 11.
